# EUROPEAN PATENT APPLICATION

(11) **EP 2 492 352 A1**
(43) Date of publication of application: **29.08.2012**
(21) Application number: 09850518.3
(22) Date of filing: 22.10.2009
(51) Int. Cl.: C12Q 1/68, C12N 9/96

(54) **COMPOSITION, METHOD AND KIT FOR DETECTING BACTERIA BY MEANS OF SEQUENCING**

(71) Applicant: BIOTOOLS BIOTECHNOLOGICAL & MEDICAL LABORATORIES, S.A., 28021 Madrid (ES)
(72) Inventor: MINGORANCE CRUZ, Jesús, E-28046 Madrid (ES); CASTÁN GARCÍA, Pablo, E-28021 Madrid (ES); FRANCO DE SARABIA ROSADO, Pedro Manuel, E-28021 Madrid (ES)
(74) Representative: Gonzalez-Alberto Rodriguez, Natalia
(86) International application number: PCT/ES2009/000507
(87) International publication number: WO 2011/048227

(57) **Abstract**

Composition, method and kit for detecting bacteria by means of sequencing, describes a method for detecting the presence and type of a microorganism present in a sample by means of stabilization and sequencing techniques and subsequent analysis of microsequences in genes encoding the ribosomal RNA most conserved, and on specific areas of the 16-S region with taxonomic value.

## Description

### Technical Field of the Invention

The present invention describes a method for detecting the presence and type of microorganism present in a sample by means of stabilization and sequencing techniques, and subsequent analysis of microsequences in genes encoding the ribosomal RNA most conserved, and on specific areas of the 16-S region with taxonomic value.

### Background of the Invention

Septicemia, or sepsis, is a set of clinical symptoms characterized by the presence and dissemination of bacteria in the blood. It is a severe, potentially fatal infection that quickly deteriorates and can result from infections in the entire body, including infections with a focal spot in the lungs, abdomen and urinary tracts. It can appear before or at the same time as bone infections (osteomyelitis), central nervous system infection (meningitis) or infections of other tissues, and in these cases it is a potentially fatal condition in people with an impaired immune system. The most severe form is referred to as septic shock, and it is a type of progress of the set of clinical symptoms that is reached when the bacterial load causes a sustained reduction of blood pressure which prevents the correct supply of oxygen to tissues.

It is estimated that 50,000 new cases of severe sepsis occur every year in Spain, and that 17,000 people die every year due to this infection. 18 million cases occur worldwide every year, and 1,400 people die every day worldwide as a result of this general infection, putting its morbidity ahead of diseases with a large social impact such as breast cancer or AIDS.

The fast diagnosis and early identification of the microorganism causing the infection allow the administration of a necessary early therapy targeting the microorganism found, which is crucial for reducing the severity of the disease and even for assuring the survival and recovery of infected patients. In this sense, there is a direct correspondence between the early diagnosis and identification and the complete total absence of sequelae associated with this set of clinical symptoms.

Hemoculture is the traditional method most widely used in the identification of infectious microorganisms, but it has the drawback of taking between one and five days in giving a precise result. In the case of infections caused by fungi, a diagnosis by means of hemoculture can take more than eight days. In summary, hemoculture is an effective but slow method of diagnosis for determining the infectious microorganism and, accordingly, the suitably therapeutic prescription for early treatment of the infection. Diagnostic sensitivity is another additional problem with diagnostic techniques associated with hemoculture. This lack of sensitivity can also be seen in the method of diagnosis by means of immunoprecipitation, which is further affected by seasonal changes in surface antigen patterns for certain pathogenic germs. Furthermore, these techniques based on biochemical and phenotypic characteristics of microorganisms often fail when applied to clinical variants due to morphological changes or changes in the metabolic state of the pathogenic microorganism at a specific time.

In fact, traditional laboratory methods for identifying microorganisms cannot always identify multiple pathogenic agents in a single clinical sample, because identification from a culture is based on the predominance of the microorganism and can be affected by the positive-negative selection thereof in the culture medium. There is evidence confirming the presence on multiple occasions of more than one microorganism per clinical sample, these polymicrobial clinical symptoms being very difficult to characterize by means of traditional culture methods. The method object of this invention is suitable for detecting several pathogenic agents in a single clinical sample regardless of the proportions of each of them.

Out of the different techniques existing for identifying and characterizing pathogenic microorganisms, techniques based on the genetic material thereof, DNA (deoxyribonucleic acid) and/or RNA (ribonucleic acid), are becoming increasingly more important. There are currently several nucleic acid hybridization assays which can be used to identify microorganisms. Out of these assays, the most widely used include nucleic acid amplification by means of polymerase chain reaction (PCR) and reverse transcriptase together with subsequent cDNA transcript amplification (RT-PCR- Reverse Transcriptase Polymerase Chain Reaction), real time nucleic acid amplification or quantitative PCR (qPCR, also called Real Time PCR), LCR (Ligase Chain Reaction Nucleic Acid Amplification), liquid phase hybridization (LPH), and in situ hybridization, among others. These assays for the specific identification of a specific microorganism use different types of nucleotide hybridization probes primarily labeled with non-radioisotopic molecules such as the digoxigenin, biotin, fluorescein or alkaline phosphatase, for the purpose of generating a detectable signal when specific hybridization between the nucleotide hybridization probe and the specific sequence of the genetic material, DNA and/or RNA, identifying the microorganism to be identified takes place. Another alternative associating the diagnosis of infection with identification of the pathogen and its particularities is the PCR-RFLP method (Restriction Fragment Length Polymorphism), referring to specific nucleotide sequences in DNA which are recognized and cut by restriction enzymes usually generating different distance, length and arrangement patterns in the DNA of different pathogens within a polymorphic population for these restriction fragments.

Among the mentioned techniques, the most widely used process is PCR (Saiki et al., Science, 230, 1350-1354 (1985), Mullis *et al*., United States patents US 4,683,195, US 4,683,202 and US 4,800,159). This technique allows serial exponential nucleic acid amplification. Said amplification is achieved by means of repetitive denaturation cycles by heat of the nucleic acid under study, binding of complementary primers to two opposing regions of the nucleic acid to be amplified, and extension of the sequence limited between the two primers within the nucleic acid by the action of a heat-stable polymerase enzyme. The repetition of successive cycles in this process achieves exponential amplification of the nucleic acid under study. Likewise, this process does not generate a detectable and intrinsic signal, so the analysis of the presence of the amplified nucleic acid requires an additional analysis of the products generated in the presence of an intercalating agent, generally by means of agarose or acrylamide gel electrophoresis.

For the purpose of avoiding this detection step on different supports, a PCR variant, real time PCR or quantitative PCR, has been developed where amplification and detection processes occur simultaneously, without the need for any further action. Furthermore, by means of detecting the amplified fragments by fluorescence, the amount of DNA synthesized at all times for the amplification can be measured, because the emission of fluorescence occurring in the reaction is proportional to the amount of DNA formed, which allows knowing and recording at all times the kinetics of the amplification reaction (Higuchi R, Fokler C, Dollinger G, Watson R. Kinetic PCR analysis: Real-time monitoring of DNA amplification reactions. Bio/Technology 1993; 11: 1026-30).

The simultaneous detection and/or identification of species of microorganisms in a specific sample requires using different nucleotide probes, different primers in the case of PCR, or different fluorescent primers and/or probes in the case of real time PCR. They must all be specific for each of the microorganisms to be detected, it normally being necessary to perform different assays for identifying each of the microorganisms in question. This need for using different probes and primers specific for identifying each of the microorganisms possibly present in the sample complicates both the experimental development of the probes or primers to be used and the viability and cost of the assay for identifying multiple microorganisms in one and the same sample. For this reason, multiplexed systems have not achieved an actual implementation in routine diagnosis.

In the case of PCR, sometimes it is possible to design consensus primers capable of amplifying a specific region of the DNA that is common to several microorganisms. In the case of eubacteria, consensus primers binding to one or more highly conserved regions of bacterial DNA, for example, the highly conserved 16S region of ribosomal RNA (rRNA) or the 23S region of the same ribosomal DNA, are well-known. The corresponding templates can be amplified using suitable consensus primers and the bacterial DNA product of this amplification can then be detected by means of different methods of detection (Anthony, Brown, French; J. Olin. "Rapid Diagnosis of Bacteremia by universal amplification of 23S Ribosomal DNA followed by Hybridization to an Oligonucleotide Array". Microbiol. 2000, pp. 781-788, and patent application WO 00/66777).

The standard methods of detection by means of amplification on a biological sample for identifying bacteria may not always identify multiple pathogenic agents present in the same sample, given that precise identification by means of amplification depends on the use of primers specific for each of the species present in the sample, which entails prior knowledge or supposition of the species present. In the case of using generic primers in the amplification reaction, identification is based on the predominance of the organism and can be affected by the prevalence of one bacterium on another. There is evidence that in a number of clinical symptoms more than one organism can be present in the analytical sample, making detection by traditional methods very difficult. The method object of this patent is suitable for detecting several pathogenic agents in a single clinical sample simultaneously without prior knowledge or supposition of the bacterial species present in the sample.

Advances in recombinant DNA molecular biology and handling techniques have lead to the development of new methods for the rapid identification of multiple pathogenic agents in a single assay from a specific biological sample over the past few years. Some of these simultaneous multifactorial analytical methods (multiplexing) are described below. Klausegger et al. ("Gram-Type Specific Broad-Range PCR Amplification for Rapid Detection of 62 Pathogenic Bacteria", J. Clin. Microbiol. 1999, pp. 464-466) show that the DNA / RNA of Gram-positive bacteria can be amplified specifically in a PCR reaction using universal primers designed especially for this group of bacteria, and that Gram-negative bacteria are not amplified in this PCR reaction, such that it is at least possible to sub-divide the eubacteria object of the investigation into Gram-positive and Gram-negative. Klausegger uses conventional microbiological methods for more precisely specifying the Gram-positive bacteria that have been amplified in this manner. According to Klausegger, through this treatment it is at least possible to rapidly treat pathogenic bacteria, the treatment targeting Gram-positive or Gram-negative bacteria based on the detection profile. However, the Klausegger publication does not allow any more precise identification within a short time period, generating a deep non-definition from the medical practice point of view when limiting treatment for patients with polymicrobial infection.

The simultaneous detection and/or identification of species of microorganisms in a given sample has recently been reported with the technique of multiplex PCR of multiple primers for nonoverlapping amplifications, with multiple groups of species-specific oligonucleotide pairs and probes corresponding to the different amplification objectives (Corless, C. E., M. Guiver, R. Borrow, V. Edwards-Jones, A. J. Fox, and E. B. Kaczmarski. "Detección simultánea de Neisseria meningitidis, Haemophilus influenzae y Streptococcus pneumoniae en casos sospechosos de meningitis y septicemia usando PCR en tiempo real". 2001. J. Clin. Microbiol. 39: 1553-1558).

The use of multiplex PCR for detecting antibiotic resistance genes has also been described (J. Clin. Microbiol, 2003, 4089-4094, and 2005, 5026-5033). The sensitivity of this assay (100 pg correspond to 10 Staphylococcus cells, useful in the direct detection of positive cultures) and the specificity thereof, requires, however, improvements in processes that increase their diagnostic value as it is located under the clinical horizon.

Currently there are several multiplexing methods, but each entails different limitations. In the multiplex detection of bacteria by means of analyzing the melting temperatures of amplified nucleic acid chains (melting curves), two oligonucleotide primers are used per bacterium and each bacterium is differentiated according to the size and composition of nucleotide bases of the amplified sequence. An important limitation of this assay is that if there are two bacteria present, or different levels of target bacteria present, the method does not work correctly. Amplification follows a non-linear profile under these conditions and false positives and negatives that are an intrinsic problem of this method are generated.

Other forms of multiple detection of bacteria use amplification of conserved regions by means of universal primers. An example of use of this method can be found in patent US 6,699,670. This method allows a rapid identification of the agents causing infection. To identify the particular microorganism causing the infection, other analyses are necessary given that, *a priori,* the identity of the microorganism is not known, and accordingly the specific probes to be used for individual identification are unknown. Therefore, the number of specific probes of each bacterial species that can be included in an individual reaction (including the universal probe and positive control) is restricted by the enzymatic kinetics of the amplification reaction and subsequent generation of the measurable identification signal. This method also suffers too many false positives and false negatives due to the stoichiometry of the hybridization process.

Other methods describe the possibility of performing, after amplification obtained using universal primers, multiple detections by means of using universal probes hybridizing with highly conserved regions for the purpose of detecting the presence of microorganisms, to subsequently use species-specific probes hybridizing to non-conserved regions of the nucleic acids extracted from the sample for the purpose of individually identifying each of the microorganism precisely, such that false positives and negatives are prevented to a great extent. An example of these methods can be found in patent application WO 2009/049007.

The regions of higher taxonomic value in relation to approaches of this type include the sequence of the 16S eubacterial ribosomal RNA (16S rRNA) gene located between positions 1671 and 3229 of the H chain of mitochondrial DNA. This gene has highly conserved regions, virtually identical for all microorganisms, and divergent regions which are different for the different species of microorganisms. This 16S gene is present in multiple copies in the genomes of all human bacterial pathogens belonging to the *Eubacteria* kingdom, and many bacterial species contain up to seven copies of the gene. A target gene that is present in multiple copies increases the possibility of detecting the infectious pathogens when they are at a low proportion or diluted in a set of clinical symptoms of polymicrobial infection. Given that the entire 16S rRNA sequence is available, and these data indicate the highly conserved nature of the gene within the *Eubacteria* kingdom, this region is a generic identification target for the components of any eubacterial genus. Furthermore, there is sufficient variation in other internal and neighboring regions of the 16S rRNA gene to provide specific discrimination between the species of the main agents causing infectious clinical symptoms such as meningitis and different varieties of septicemia. For this reason, the fact that this widely studied gene is used by default in the identification and characterization of eubacteria. Different descriptions of the use of this ribosomal gene can be found in Weisburg W G., Barns S.M., Pelletier D., Lañe D. "16S Ribosomal DNA Amplification for Phylogenetic Study" Journal of Bacteriology, Jan. 1991, Vol. 173 p. 697-703; Case RJ, Boucher Y, Dahllöf I, Holmstróm C, Doolittle WF, Kjelleberg S. "Use of 16S rRNA and rpoB genes as molecular markers for microbial ecology studies". January 2007. Appl. Environ. Microbiol. 73 (1): 278-88; and in Coenye T, Vandamme P "Intragenomic heterogeneity between multiple 16S ribosomal RNA operons in sequenced bacterial genomes". November 2003. FEMS Microbiol. Lett. 228 (1): 45-9.

For the purpose of being able to distinguish between species or showing genetic variations such as the presence of pathogenicity factors within one and the same bacterial species, obtaining the nucleotide sequence from its genetic material is in many cases the only possibility of differentiation. This nucleotide sequence can be obtained using conventional sequencing guidelines based on the process described by Sanger and Coulson in 1975 (Sanger F, Coulson AR. "A rapid method for determining sequences in DNA by primed synthesis with DNA polymerase". J Mol Biol. May 25, 1975, 94 (3) :441-448 and Sanger F, Nicklen s, and Coulson AR, "DNA sequencing with chain-terminating inhibitors", Proc Nati Acad Sci USA. 1977 December; 74(12): 5463-5467). These methods are based on using dideoxynucleotides (ddNTPs) lacking one of the hydroxyl groups, such that when one of these nucleotides is incorporated in a growing DNA chain, this chain cannot continue to elongate since the DNA polymerase enzyme used to perform elongation needs a 3' OH end to add the next nucleotide and the incorporated deoxynucleotide lacks this hydroxyl group. Subsequently, electrophoretic processes in gel or by means of automated capillary electrophoresis are used to resolve the nucleotide sequence triplets incorporated for each dideoxynucleotides species. These electrophoretic processes are difficult to introduce in clinical practically primarily because of their economic cost and the time required for analysis.

An example of applying conventional sequencing based on the Sanger method in the identification of bacteria can be found in Fontana, et al. "Use of MicroSeq 500 16S RNA Gene-Based Sequencing for Identification of Bacterial Isolates that Commercial Automated Systems Failed to Identify", 2005. J Clin. Micr. Vol. 43, No. 2.

The high demand for low-cost sequencing that has been generated from the consideration of initiative such as the Human Genome Project and its derivations to other animal, plant and microbial models, has primarily led to new sequencing technologies. One of these new sequencing technologies is referred to as "sequencing by synthesis", which uses the DNA synthesis process by means of DNA polymerase to identify the bases present in the complementary DNA molecule. Basically, the different sequencing by synthesis methods developed until now consist of labeling the oligonucleotide primer or the terminators with a fluorescent compound for subsequently activating the sequence reaction. The reaction products are directly detected during electrophoresis when passing in front of a laser which allows detecting the emitted fluorescence by exciting fluorophores.

One of the most widely used sequencing by synthesis methods is pyrosequencing, a technique which uses DNA polymerase enzyme-dependent DNA polymerization to polymerize nucleotides in sequence. The process is completed by incorporating a different type of deoxynucleotides every time for detecting and then quantifying the number and species of nucleotide added to a specific location by means of the light emitted in the release of pyrophosphates (byproducts of extension by polymerization of the DNA chain). Descriptions of this technique can be found in M. Margulies, et al. "Genome sequencing in microfabricated high-density picolitre reactors". 2005. Nature 437, 376-380 and in M. Ronaghi, S. Karamohamed, B. Pettersson, M. Uhlen, and P. Nyren "Real-time DNA sequencing using detection of pyrophosphate release". 1996. Analytical Biochemistry 242, 84:89. Methods of sequencing using pyrosequencing can be found in patent applications W01998/028440 and W02000/043540, assigned to Pyrosecuencing AB, and W02005/060345, assigned to Biotage AB. Due to the limitations inherent to the technique, pyrosequencing only allows sequencing short DNA fragments with a maximum of between 50 and 80 nucleotides per completed reaction.

Pyrosequencing is beginning to be a widely used technique to identify the species to which bacteria belong, the presence of which bacteria is suspected or has already been previously verified by other methods of identification, such as real time PCR. Its application on clinical samples even allows identifying pathogenicity factors and/or antibiotic resistance of specific bacteria, but there is currently no method of pyrosequencing that can be used in the taxon-specific identification of bacteria from clinical samples, food samples or environmental samples, without having prior knowledge of the generic group to which the bacterium/bacteria found in that sample belong, and for the simultaneous identification of several bacteria present in a sample without previously knowing or suspecting which bacteria can be found in it.

Basically, since its origin pyrosequencing has been aimed at the massive but not focused analysis of more or less short sequences within a mixture of samples of DNA to identify. In fact, the journal Science has recorded within its list of most relevant advances of the year 2008 the extraction of more than one million DNA bases of a Neanderthal fossil, 13 million bases of the genome of a mammoth, and more than 15 million bases of the DNA of bacteria, fungi, viruses and plants of the period in which those animals lived, using new techniques of analysis such as metagenomic technique and pyrosequencing. For this reason, the focused analysis approach taxon-specific for any eubacterial species by means of pyrosequencing seeks a new application in a less advanced field for this technique.

Different examples of the focused use of this technique in the field of identifying microorganisms are listed below by way of reference: Brown-Elliott BA, Brown JM, Conville PS, Wallace RJ Jr. "Clinical and laboratory features of the Nocardia spp. based on current molecular taxonomy". Clin Microbiol Rev. 2006 Apr; 19(2), 259-82; Jalava J and Marttila H. "Application of molecular genetic methods in macrolide, lincosamide and streptogramin resistance diagnostics and in detection of drug-resistant Mycobacterium tuberculosis". APMIS. Dec 2004, 112(11-12): 838-855; Clarke SC. "Pyrosequencing: nucleotide sequencing technology with bacterial genotyping applications". Expert Rev Mol Diagn. 2005 Nov. 5(6):947-53; Ronaghi M., Elahi E. "Review Pyrosequencing for microbial typing". Journal of Chromatography B. 2002, 782 67-72; Engstrand L. "The usefulness of nucleic acid tests for the determination of antimicrobial resistance". Scandinavian Journal of Clinical and Laboratory Investigation. 2003, Volume 63, Supplement 239: 47-52.

An example of use of sequencing by synthesis using the 16S gene can be found in J. A. Jordan, A. R. Butchko, M. B. Durso "Use of Pyrosequencing of 16S rRNA Fragments to Differentiate between Bacteria Responsible for Neonatal Sepsis.", Journal of Molecular Diagnostics, February 2005, Vol. 7, No., 105-110. Another example of use can be found in patent WO/2004/046385 "A method for inter-species differentiation and identification of a gram-positive bacteria", assigned to Biotage AB.

Another example of the use of sequencing by synthesis in the identification of microorganisms in a sample can be found in patent US 2004023209, assigned to Pyrosequencing AB. This patent describes a process of identifying bacteria using degenerate or low-specificity oligonucleotides as primers of the amplification reaction by means of the nucleic acid amplification process referred to as "primer extension" which amplify non-specific regions of bacterial 16S and CoNS genes instead of the specific oligonucleotide primers of specific regions of the 16S gene described in the present invention. The nucleotide fragments obtained by means of using degenerate primers have different sizes, and these different sized fragments are subsequently sequenced, the sequences obtained being analyzed to identify the microorganism in question at the species level.

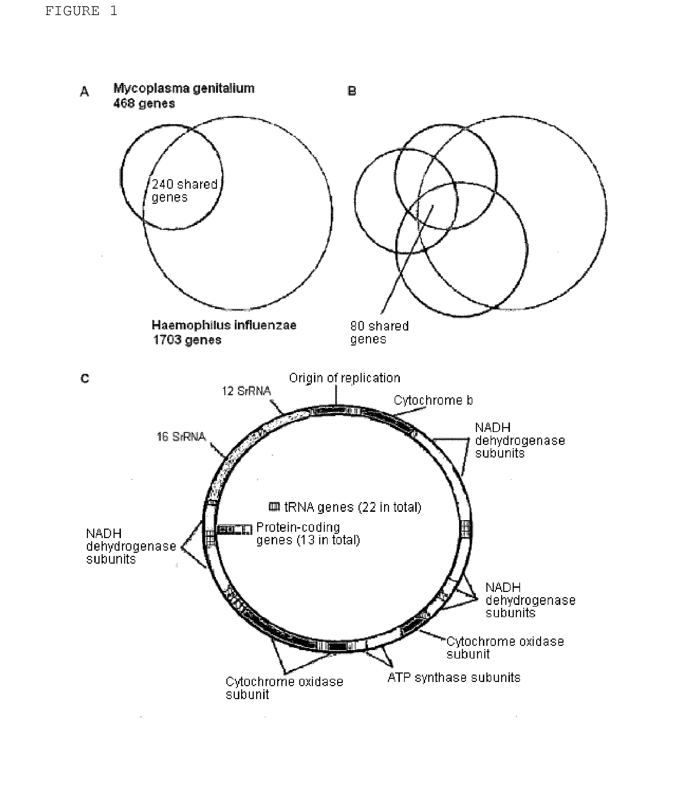

An example of the comparison of relative homologies between M. *genitalium* and *H. influenzae* (240 common genes), as well as with 25 bacterial groups [not all of them are drawn, only the genome overlap area] selected randomly (80 genes). In all cases, the ribosomal genes on which this invention is based are conserved and adopt a relative distribution according to the diagram in Figure 1. (C): Relative distribution, according to a circular diagram, of the genome overlap area corresponding to the first superposition of the preceding figure. The arcs corresponding to ribosomal genes have a length proportional to the total of the homology among the analyzed species (M. *genitalium* and *H. influenzae*)*.*

In nucleic acid amplification by means of these techniques (amplification which is also used in sequencing by synthesis processes), each of the components involved in the reaction, i.e., the DNA polymerase enzyme, the reaction buffer with the reaction-enhancing additives or stabilizers, magnesium chloride, or manganese chloride in the case of RT, oligonucleotides used as reaction primers, deoxyribonucleotides (dATP, dCTP, dGTP and dTTP) and the sample containing the nucleic acid to be amplified, are separate from one another, conserved by means of freezing, and must be mixed prior to performing the reaction, it being necessary to add and mix very small amounts (microliters) of each of them. This action produces frequent errors in the administration and pipetting of each of the mentioned reagents, which ends up generating uncertainty as to the reproducibility of the results obtained by means of applying these techniques, particularly preoccupying uncertainties in the case of human diagnosis. This variability due to the possibility of error in pipetting the different reagents to be added to the amplification reaction also affects the sensitivity of the technique, which generates a new uncertainty concerning the application of these techniques in the human diagnosis of diseases, and especially in the determination of levels of infection and of levels of gene expression.

Furthermore, for pipetting and adding the sample to be analyzed to the reaction mixture aerosols are produced which frequently cause cross-contaminations between samples to be analyzed (Kwok, S. et al., Nature, 1989, 339:237 238), generating false positive results, which are extremely important in the case of human diagnosis.

Different systems of preparing and stabilizing enzymatic activities have been developed for the purpose of preventing errors inherent to the excessive manipulation commonly required for use thereof, as well as to eliminate problems of cross-contaminations. Patent application WO 93/00807 describes a system for stabilizing biomaterials for the lyophilization process. Other references are the following patents and documents: US 5,861,251 assigned to Bioneer Corporation; WO 91/18091, US 4,891,319 and US 5,955,448, assigned to Quadrant Holdings Cambridge Limited; US 5,614,387, assigned to Gene-Probe Incorporated; US 5,935,834, assigned to Asahi Kasei Kogyo Kabushiki Kaisha, and publications: Pikal M.J., BioPharm 3:18-20, 22-23, 26-27 (1990); Carpenter et al., Cryobiology 25:459-470 (1988); Roser B., Biopharm 4:47-53 (1991); Colaco et al., Bio/Technol. 10:1007-1011 (1992); and Carpenter et al., Cryobiology 25:244-255 (1988).

Biotools Biotechnological & Medical Laboratories, S.A, author of the present application, has developed a system of stabilization by means of gelling complex mixtures of biomolecules which allows stabilizing reaction mixtures for long periods of time in widely varying storage conditions (WO 02/072002). Complex reaction mixtures, such as mixtures for gene amplification reactions, containing all the reagents necessary for performing the experiment, aliquoted in independent ready-to-use vials have been stabilized by means of using this system, in which it is only necessary to reconstitute the reaction mixture and add the test nucleic acid.

In summary, there is currently a growing demand for diagnostic methods capable of identifying bacteria in a rapid and precise manner in clinical samples, preventing cross-contaminations and simplifying complex human manipulation generally required for the previously described molecular methods, substantially improving the reproducibility and reliability of the diagnostic results obtained. In clinical emergency situations caused by septicemia, the correct, rapid and precise identification of the bacterium or bacteria present and/or causing the infection or potential future infection is a first-order need in medical practice for prescribing the best and most suitable treatment available. This need for a rapid, precise, reproducible and easy to perform diagnosis is currently not met.

The purpose of the present invention is to meet this need for a taxon-specific simultaneous, rapid, precise, reproducible and easy to perform identification of the bacteria present in a clinical sample when the genus of bacteria present in the sample is not previously known by means of the method and kit objects of the invention.

### Description of the Drawings

Figure 1. (A and B): Comparison of relative homologies between M. *genitalium* and *H. influenzae* (240 common genes), as well as with 25 bacterial groups [not all of them are drawn, only the genome overlap area] selected randomly (80 genes). In all cases, the ribosomal genes on which this invention is based are conserved and adopt a relative distribution according to the diagram in Figure 1. (C): Relative distribution, according to a circular diagram, of the genome overlap area corresponding to the first superposition of the preceding figure. The arcs corresponding to ribosomal genes have a length proportional to the total of the homology among the analyzed species (M. *genitalium* and *H. influenzae*)*.*

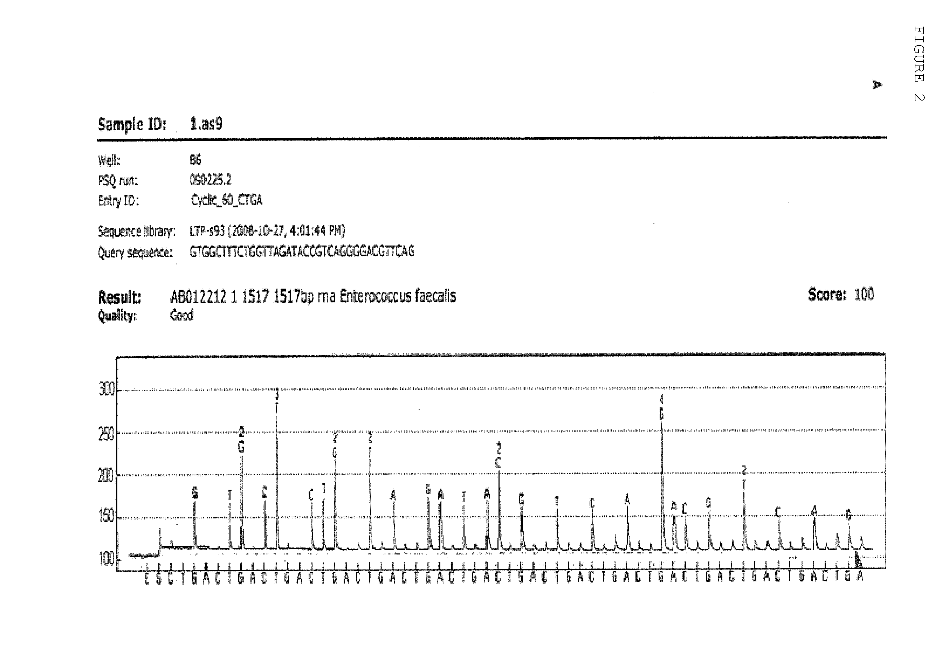

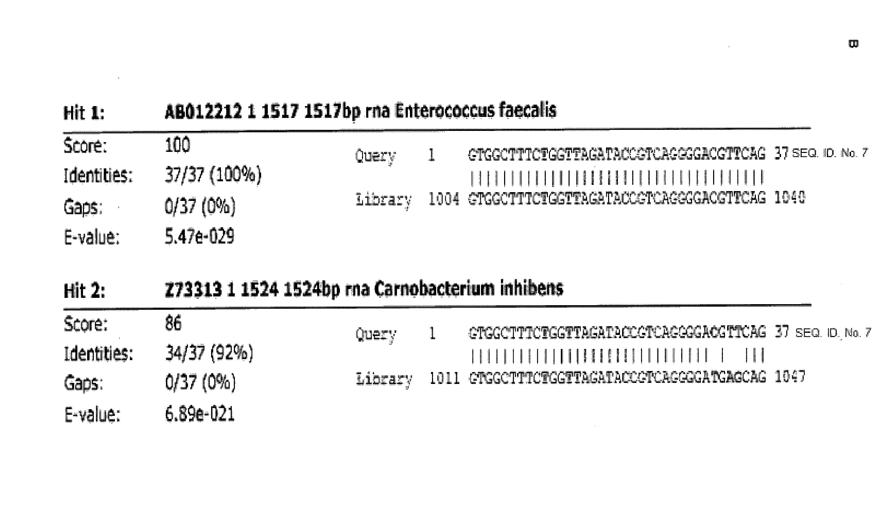

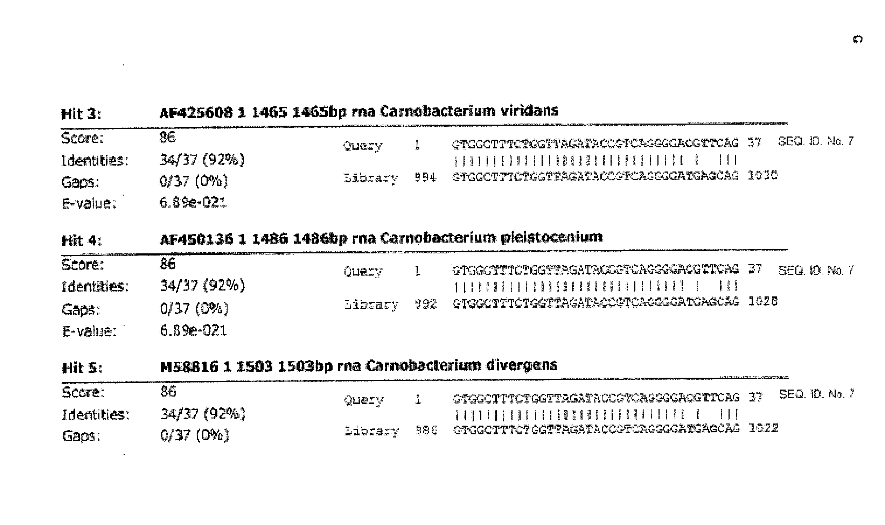

Figure 2: Results of the pyrosequencing reaction relating to Example 1.

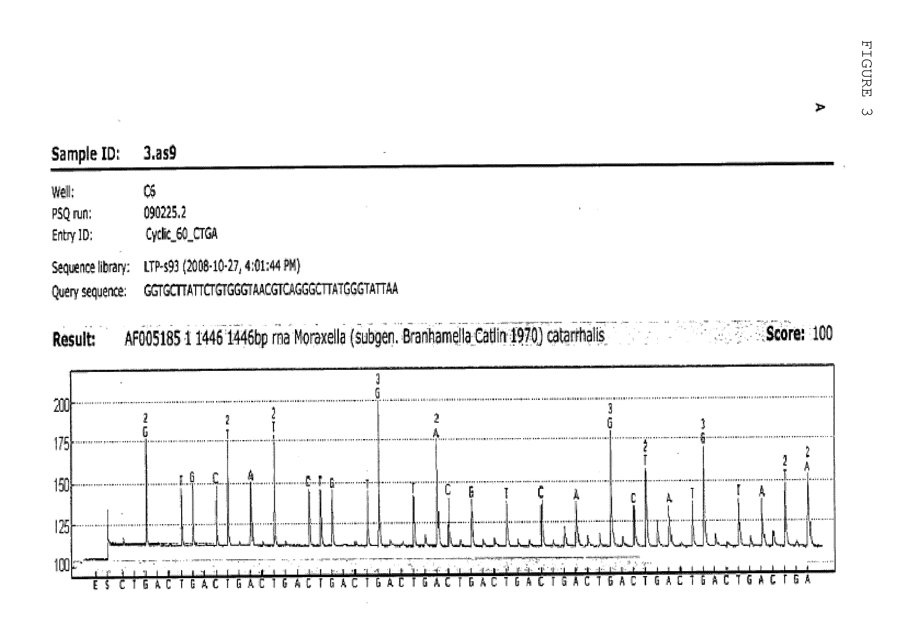

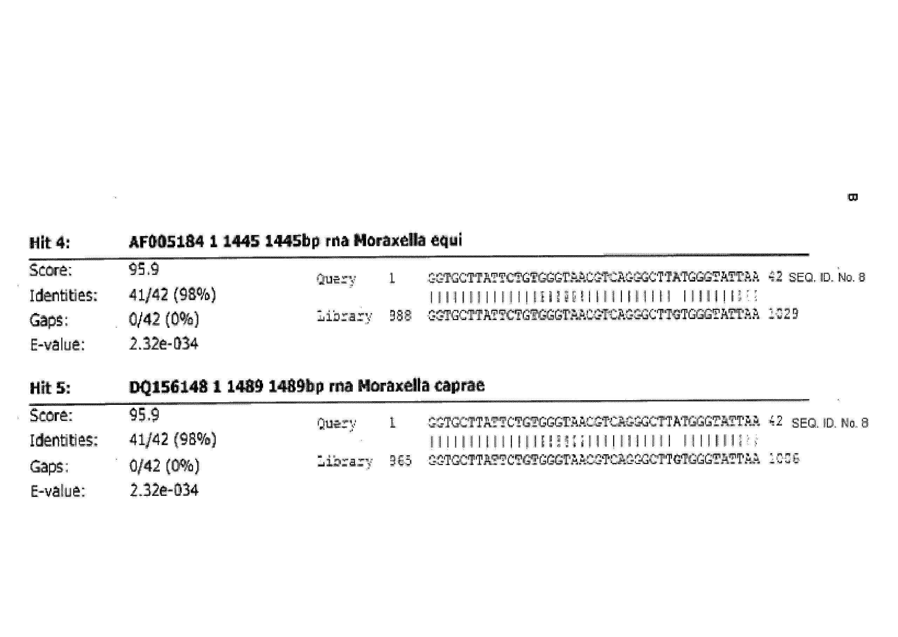

Figure 3: Results of the pyrosequencing reaction relating to Example 2.

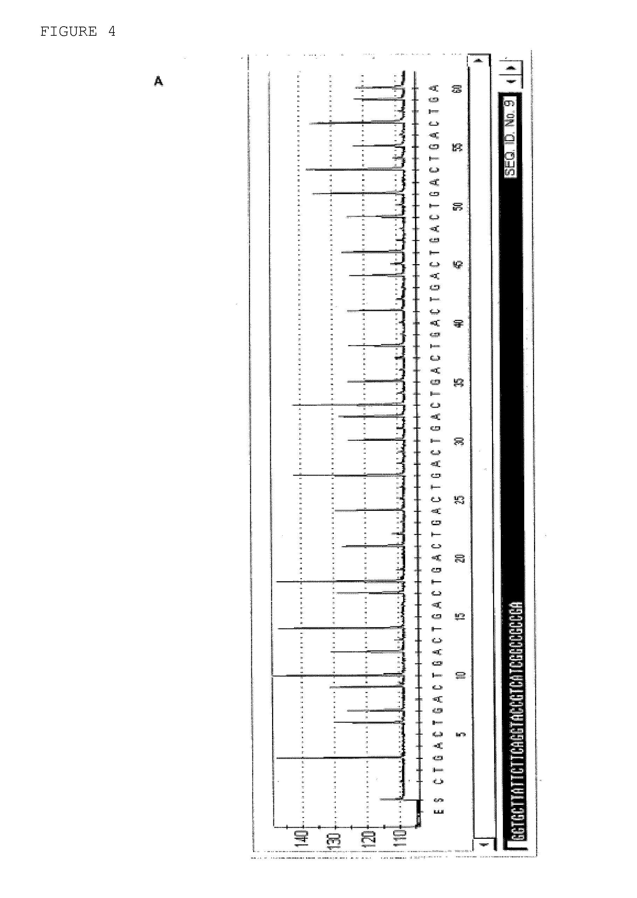

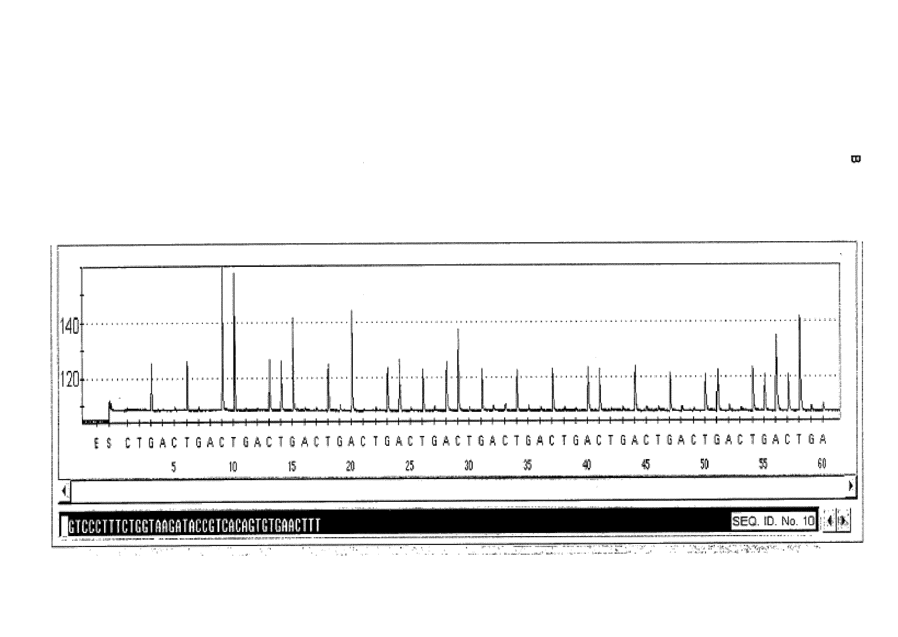

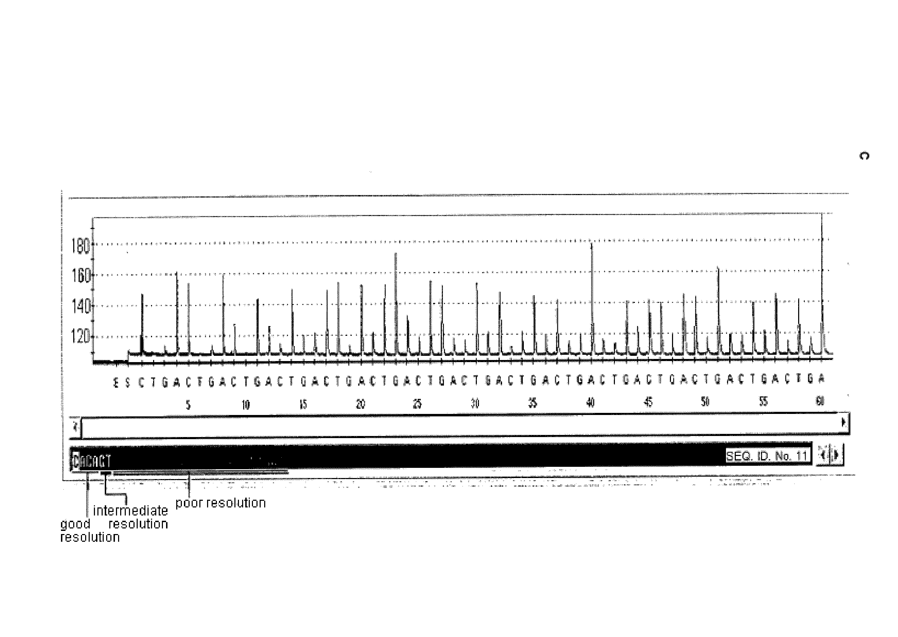

Figure 4: Quality of pyrosequencing using gelled amplification reagents. (A) On a 36-base sequence obtained, all were considered to have good resolution, no possible indeterminacy being recorded. (B) On a 38-base sequence, as in Figure 4a, no indeterminacy was recorded in 38 sequenced bases. (C) On a 33-base sequence, in this case only 4 were considered to have good resolution (bases in light gray), two were considered to have intermediate resolution (bases in white), and 27 were considered as possible indeterminacies (bases in dark gray).

### Object of the Invention

The present invention consists of a new rapid, precise and easy-to-handle method for differentiating and identifying bacteria in a biological sample by means of the sequencing analysis (sequencing by synthesis method) of three regions of the 16S bacterial ribosomal RNA gene, obtaining a taxon-specific genetic pattern based on the nucleotide sequence obtained, as well as the kit containing the reagents necessary for carrying out said method which are stabilized by gelling and subsequently dried to a degree of humidity of 10% to 30%. To perform this taxon-specific identification, prior knowledge or supposition of the family, genus or species of the bacterium or set of different bacteria contained in the sample is not necessary. The sequencing method used is preferably sequencing by synthesis, and within the different sequencing by synthesis methods which are used today, the method preferably used is pyrosequencing, although any other nucleic acid sequence analysis method currently used or to be used in the future could be used.

The sequences nucleotides identifying each of the bacteria are well-known and are available in different published genetic databases, such as GenBank and EMBL, among others.

A rapid and reliable diagnostic result with very little manual manipulation is obtained by means of using pyrosequencing and stabilization by means of the gelling process of the different reagents involved in pyrosequencing in each of the wells of the plates in which the analysis is performed.

Furthermore, as stabilization is performed by means of the gelling process by adding to the medium a stabilizing aqueous solution and subsequently drying to a degree of humidity between 10% and 30%, the unexpected advantage of an improvement in resolution between 75% and 90%, even in normal sequence sizes (30-50 bases), is surprisingly obtained. As a result of said advantage sequencing of fragments which are between 20% and 35% longer than those obtained with methods that do not include said stabilization can also be obtained.

The present invention further allows in a single analysis the multiple identification of the different bacteria that may be present in the biological sample, without needing to have prior knowledge or supposition of the type of bacteria that may be present, using to that end only three primer pairs in the amplification of the fragment to be sequenced, the taxonomic value of which allows overlapping the information generated by each sequence, advancing in the process of identifying the levels from family to genus and finally to species.

The present invention additionally allows discarding false negatives from hemocultures. For example, Table 1 shows the percentage of results successfully identified at the species, genus and family level on 60 samples of positive hemoculture after incubation and the percentage of results successfully identified at species, genus and family level on 12 samples of negative hemoculture after incubation.

**TABLE 1: percentage of results successfully identified at the species, genus and family level**

| % identification | Family taxon | Genus taxon | Species taxon |
|---|---|---|---|
| Positive hemoculture | 100% | 99% | 96% |
| Negative hemoculture | 45% | 45% | 40% |

Due to the great sensitivity of the analysis, it is necessary to use an ultrapurified DNA polymerase enzyme (free of any contamination with bacterial DNA that may be generated during the biological enzyme synthesis process) in the sequencing by synthesis step, since the presence of this contaminating DNA can lead to false positives that would modify the result of the diagnosis (C.E. Corless, J. Clin. Microbiol, 2000, 1747-1752). This possible contamination is particularly important when the DNA polymerase enzyme has been synthesized as recombinant in *Escherichia coli,* since the bacteria forming the taxonomic group to which *E*. *coli* belongs (Gram-negative γ-proteobacteria) are the primary pathogens to be identified in the case of septicemia. Furthermore, given the evolutionary proximity existing between them, the exponential increase in the number of copies inherent to nucleic acid amplification requires the use of a high copying fidelity DNA polymerase enzyme for the purpose of preventing the introduction of isolated mutations in initial amplification cycles that may falsify the obtained sequence. This alteration of the obtained sequence would entail an erroneous result of the analysis. The kit described in this patent incorporates the Ultratools DNA polymerase enzyme manufactured by Biotools Biotechnological & Medical Laboratories S.A. to prevent this problem.

More specifically, the present invention consists of a process requiring an initial generic extraction step by means of standardized, manual or automated techniques followed by a process of initial amplification of the most conserved ribosomal DNA, allowing by means of using three different and simultaneous amplification reactions, serial superposition of the results generated by the sequencing thereof to reach taxonomic levels of identification at the genus, family and species level. The purpose of this initial amplification of the selected ribosomal regions is to generate fragments labeled by biotinylation at the 3'OH end, which will subsequently be immobilized, isolated by basic denaturation of the generated double helix, and finally sequenced. This process of amplification is performed in a multiwell plate in which each of them contains all the reagents necessary for performing the amplification specific for the subsequent sequencing process, i.e., Biotools high copying fidelity ultrapure DNA polymerase, biotinylated primers described in the present invention, deoxynucleotides to be incorporated in the amplification reaction (dATP, dCTP, dGTP, dTTP), and the optimized reaction buffer. All these reagents are stabilized by means of gelling according to the process described in patent WO 02/072002, such that it is only necessary to add bidistilled water and the nucleic acid extracted from the sample in an initial generic extraction step to perform this amplification because the remaining necessary reagents are already previously dispensed on the multiwell plate at the precise concentrations required.

The biotinylated products obtained by means of the previous amplification reaction form the substrate for the immediately following pyrosequencing reaction. These biotinylated products are purified prior to being transferred to the second plate in which the process of pyrosequencing is carried out, using the method and instruments recommended by the manufacturer of the apparatus used for sequencing by synthesis. The purification process is performed in three steps and only requires a dispensing system connected to a vacuum pump, as is described by the manufacturer of the apparatus used for pyrosequencing.

This second plate contains in each well all the reagents necessary for carrying out the pyrosequencing reaction on each of the fragments labeled in the previous amplification. These enzymes and reagents incorporated in each of the wells of the plate are high-fidelity ultrapure DNA polymerase, ATP-sulfurylase, luciferase, apyrase, sequencing primer, luciferin, adenosine-5'-phosphosulfate (APS), deoxynucleotides to be incorporated in the extension reaction of the DNA chain to be sequenced (dATP, dCTP, dGTP, dTTP), and the reaction buffer. All these reagents are stabilized by means of gelling as described in patent WO 02/072002, at the precise concentrations required to complete the sequencing by synthesis reaction.

The process described in this patent also incorporates the innovation of using the same primer in the sequencing by synthesis reaction as the one used in the prior amplification reaction to limit the non-biotinylated end for the initial amplification. This allows reducing the number of necessary primers to two (Amplification fwd*-biotinylated, Amplification rvs = Sequencing fwd), provided that the conditions of the sequence limited in the initial amplification are compatible with the activity of the enzymes involved in the final sequencing process (high-fidelity ultrapure DNA polymerase, ATP-sulfurylase, luciferase and apyrase).

It has been found that the components forming the gelling mixture supplied by Biotools Biotechnological & Medical Laboratories S.A. and described in patent WO 02/072002 stabilize the reagents and enzymes involved in both the amplification reaction and the subsequent pyrosequencing reaction, improving the performance of the resolution capacity, and the sequencing of oligonucleotides fragments that are longer than what is possible by sequencing when these reagents and enzymes are not stabilized by means of gelling being achieved as a result. The improvement in pyrosequencing resolution between 75% and 90% is unexpected and furthermore important for obtaining figures of up to 100% certainty in the identification of the bacteria in the sample. Sequence indeterminacy of sequence is considered the non-precise determination of the nucleotide base making up the nucleic acid sequence. When adding the stabilizing mixture, a substantial improvement in the discrimination between the emission peak corresponding to the nucleotide incorporated and the background noise caused by the remaining substrates of the pyrosequencing reaction is observed. Quality is determined by how well-defined the emission peak of a dNTP forming the sequence is, generating an intensity that clearly differentiates it from background noise and interference. For that reason, in the case of the non-gelled mixture, only the first three bases are obtained with maximum quality according to the pyrosequencing algorithm because after the third round, the background is differentiated less and less from the emission peaks corresponding to each dNTP incorporated. An example is that of two high-quality sequences in total obtained when applying gelling (Example 3, figure 4).

Specifically, the gelling mixture formed by trehalose, melezitose, glycogen or raffinose and lysine or betaine, is considered to be especially beneficial in the pyrosequencing reaction.

The sequences obtained after the sequencing by synthesis process are compared with the sequences deposited and registered in public databases for the purpose of obtaining the precise identification of the microorganisms present in the sample to be analyzed. The alignment of the generated sequences is completely compatible with the search engines typically used in clinical practice and research, being able to be done, for example, using the BLAST search engine on the GenBank (NCBI) sequence base, Assemble sequence base, etc.

The composition and reagents described can be packaged in individual kits. The kit incorporating the present invention is made up of a first multiwell plate containing in each well one of the three nucleotide primer pairs labeled at the 3' OH end by means of biotin, or any other type of labeling usable for sequencing, such as fluorophores, necessary for obtaining the labeled sequenceable fragments, together with all the reagents necessary for amplification free of contaminating DNA (high-fidelity ultrapure DNA polymerase enzyme, deoxynucleotides and reaction buffer), dosed at optimal concentrations for generating the amplification reaction, all of them pre-mixed and stabilized by means of gelling. The fragments resulting from this amplification could be sequenced by means of any known sequencing method, the sequence obtained identifying the species of bacterium or the different species of bacteria present in the sample. These fragments resulting from this amplification are preferably sequenced by means of sequencing by synthesis techniques, and more preferably by means of the technique referred to as pyrosequencing.

The sequenceable labeled fragments are obtained in this first plate described above and after purification are transferred to a second plate wherein each well contains all the necessary elements for carrying out the sequencing reaction which are pre-mixed at optimal concentrations for generating the amplification reaction, and stabilized by means of gelling. In the preferred case of using pyrosequencing, these necessary elements which are pre-mixed and stabilized are: high-fidelity ultrapure DNA polymerase, ATP-sulfurylase, luciferase, apyrase, sequencing primer (as described above), luciferin, adenosine-5'-phosphosulfate (APS), deoxynucleotides to be incorporated in the extension reaction of the DNA chain to be sequenced (dATP, dCTP, dGTP, dTTP), and the reaction buffer.

### Detailed Description of the Invention

In the present invention, "taxon-specific identification" is understood as the capacity of a specific analytical method to distinguish and identify at the taxonomic species level a specific eubacteria from several other species of microorganisms that may or may not be present in the sample to be analyzed.

"Sample" is understood as any type of sample which may potentially contain bacteria and which is possible to analyze by means of the method indicated in the present invention, either directly or indirectly, for example by means of bacterial culture from the initial sample. The sample can be a blood sample, urine sample, cerebrospinal fluid sample, sputum sample, nasal secretion sample, or any another type of body fluid or secretion, from both humans and animals, or the bacterial culture of any type or format from these fluids. The sample can also come from foods or food liquids intended both for humans and animals, or from the bacterial culture from these foods, or from environmental samples such as water, soil or air that are or are not concentrated, or the bacterial culture from said environmental samples.

"Oligonucleotide" is understood as a single-stranded polymer consisting of at least two nucleotide subunits bound to one another by means of a covalent-type bond or equivalent strong interaction. The sugar groups of the nucleotide subunits can be ribose, deoxyribose, or modifications derived from these sugars. The nucleotides units of an oligonucleotide can be bound by phosphodiester bonds, phosphothioate bonds, methylphosphoate bonds, or any another bond that does not prevent the hybridization capacity of the oligonucleotide. Furthermore, an oligonucleotide can contain uncommon nucleotides or nonnucleotide molecules, such as peptides. As it is used herein, an oligonucleotide is a nucleic acid, preferably DNA, but it could be RNA or a molecule containing a combination of ribonucleotides or deoxyribonucleotides covalently bound to one another.

The term "primer" refers to an oligonucleotide acting as a starting point of the enzymatic synthesis of DNA under conditions in which polymerization of the nucleotides occurs after the mentioned primer, extending it and introducing the nucleotides in a complementary manner into the nucleic acid chain serving as a template. This elongation of the chain takes place under suitable temperature and reaction buffer conditions. In the present invention, the primer is preferably a single-stranded oligonucleotide with a length comprised between 15 and 40 nucleotides.

In the present invention the terms "nucleic acid", "oligonucleotide" and "primer" refer to oligomer fragments consisting of nucleotides. These terms must not be limited by their length expressed in the form of nucleotides forming the linear polymer, the nucleotides forming them being deoxyribonucleotides containing 2-deoxy-D-ribose, ribonucleotides containing D-ribose, and any another N-glycoside of a purine and pyrimidine base, or of modifications of these purine and pyrimidine bases. These terms refer to single-stranded and double-stranded DNA, as well as to single-stranded or double-stranded RNA.

The term "amplification conditions" refers to the reaction conditions (temperature, buffering conditions, etc.) under which the amplification reaction of the nucleic acid template to be amplified takes place. In the present invention, the sole requirement of the amplification conditions is to maintain the annealing temperature at 54°C. The remaining parameters can be adjusted depending on the origin, extraction method and yield, without contrasted losses of robustness in the process.

"Amplification" is understood as the reaction which increases the number of copies of a specific region of a nucleic acid.

"Sequencing" is understood as any chemical, physical or enzymatic process intended for knowing the specific nucleotide sequence of a fragment of ribonucleic acid (RNA) or deoxyribonucleic acid (DNA) from a specific sample.

The term "sequencing by synthesis" refers to any nucleic acid sequencing method requiring enzymatic activity necessary for consolidating nucleotide bonds between the subunits previously described as deoxyribonucleotides, these being the functional substrates of the sequencing reaction.

"Nucleotide pattern" is understood as the product/result of sequencing, preferably of sequencing by synthesis. The nucleotide pattern represents the order in which the nucleotides are incorporated in the sequencing reaction.

"Stabilization" is understood as the preservation of chemical and biochemical qualities of the different reagents, reaction buffers, reaction enhancers, and enzymes involved in an enzymatic reaction, in this case nucleic acid amplification and reactions associated with sequencing by synthesis, once all these reagents, reaction buffers, reaction enhancers and enzymes are included in one and the same container, in this case tubes or multiwell plates, such that each of them is dosed with optimal reaction amounts and they do not interact or react with one another, immobilizing the biochemical reaction in which they are involved, being able to activate the enzymatic reaction as desired by the user, without there being a significant decrease in activity, after days, weeks, months or even years have passed after mixture and stabilization.

Stabilization thus understood is achieved by means of adding a stabilizing mixture to a solution containing the reaction mixture, and the subsequent removal of all or part of the water present in the solution resulting. This removal of all or part of the water can be achieved by means of lyophilization, fluid bed drying, room temperature and atmospheric pressure drying, ambient temperature and low pressure drying, high temperature and atmospheric pressure drying, and high temperature and low pressure drying processes.

In the present invention, the stabilization process preferably used is stabilization by means of gelling, described in patent WO 02/072002, assigned to Biotools Biotechnological & Medical Laboratories, S.A. The stabilizing mixture of the reaction mixture is preferably made up of trehalose, melezitose, lysine or betaine and glycogen or raffinose, at different concentrations regardless of the enzymatic reaction to be stabilized. The gelling mixture is more preferably made up of trehalose, melezitose, glycogen and lysine. In the present invention, the method of extracting water from the reaction mixture after adding the mixture of stabilizing agents is preferably drying by means of a vacuum at a temperature comprised between 30°C and 40°C, depending on the enzymatic reaction to be stabilized. Specifically, in the present invention the humidity content is maintained between 10-30% water.

The present invention relates to a method for performing the taxon-specific identification of one or several eubacteria simultaneously in a sample by means of the analysis of the nucleotide pattern of the nucleotide sequence or sequences obtained by means of sequencing by synthesis of three different regions belonging to the 16S ribosomal RNA gene, previously amplified before sequencing. In a first aspect, the invention is based on the possibility of identifying a bacterium, arriving at the taxonomic species level, using the nucleotide pattern obtained by the superposition of the sequences of three separate regions of the 16S ribosomal RNA gene. This nucleotide pattern represents an unequivocal genetic signature identifying the different bacterial species present in a sample and can be compared with the reference patterns deposited in different published genetic databases using search engines expressly designed for such purpose.

To carry out the invention, three different primer pairs designed for the purpose of amplifying three different regions of the 16s eubacterial ribosomal RNA gene are used (Table 2). The specific sequences of this gene are well-known and are available in several published databases, such as GenBank and EMBL. The amplification primers have been designed based on the state of the art, considering their content at the cytosine and guanine bases, as well as the multiple design alternatives that could be overlapped in the selected regions for the purpose of preventing the formation of internal secondary structures, preventing the formation of dimerizations between primers and weighting their melting temperatures to reach optimal adjustment of the nucleotide chain template. An annealing temperature standard has been established at 54°C, which could be modified according to changes between sequences to be hybridized.

**TABLE 2: Sequences of the primers used in PCR amplification of the 16S rRNA gene.**

| V1 region | | |
|---|---|---|
| Bio-VIF | Bio- SEQ. ID No. 1 | Biotin-GKAGAGTTTGATCCTGGCTCAG |
| V1 b | SEQ. ID No. 2 | GYRTTACTCACCCGTYCGCCRCT |

| V2 region | | |
|---|---|---|
| Bio-ASF | Bio- SEQ. ID No. 3 | Biotin-ACACGGYCCAGACTCCTAC |
| AS9B | SEQ. ID No. 2 | CGGCTGCTGGCACGKAGTTAGCC |

| V3 region | | |
|---|---|---|
| Bio-V3F | Bio- SEQ. ID No. 5 | Biotin-GCAACGCGAAGAACCTTACC |
| V3S | SEQ. ID No. 6 | GACGACARCCATGCASCACCT |

The first primer pair indicated in Table 2 amplifies the region of the 16S gene limited by its sequences, generating average fragment of approximately 250 nucleotide base pairs within the highly conserved ribosomal region. Since it is the largest fragment, the result of its sequence identifies the eubacteria present in the sample at a generic level, although the superposition of the sequences obtained with the following primer pairs is necessary to reach a level of identification at the species level.

The second and third primer pairs indicated in Table 2 amplify the region of the 16S gene limited by its sequences, generating an average fragment of approximately 100 nucleotide base pairs within the highly conserved ribosomal region. Depending on the degree of intraspecific variation of the eubacteria present in the sample, less superposition with one or both amplification products will be necessary, although the overlap of the three sequences (250bp + 100bp + 100bp) generates an identification percentage greater than 96%, as shown in Figures 2 and 3.

The amplification fragments obtained can be sequenced using any type of amplification reaction of specific sequences of the DNA or RNA of any one organism. In the present invention, the amplification fragments are obtained simultaneously and in the same amplification reaction by means of the PCR technique using the three primer pairs indicated in Table 2 and described above (sequences SEQ. ID. No. 2, 4 and 6). For the design of a robust process, the use of a DNA polymerase not containing traces of contaminating exogenous DNA and at the same time having a low rate of error in the incorporation of nucleotides, such as Ultratools DNA polymerase enzyme (Biotools Biotechnological & Medical Laboratories, S.A.), is virtually necessary.

The PCR amplification conditions indicated in Table 3 were optimized to achieve the reaction conditions suitable for simultaneous amplification of the three regions of the bacterial 16S gene extracted from the sample. Each amplification fragment can also be amplified in PCR reactions performed separately. In the present invention, the amplification is performed in a single reaction, whereby the three regions that will subsequently be sequenced to identify the bacterial species present in the sample are simultaneously amplified.

**TABLE 3: Exemplary table listing the amplification conditions. (The sole requirement of the amplification is to maintain the annealing temperature at 54°C, the remaining parameters can be adjusted depending on the origin, extraction method and yield, without contrasted losses of robustness in the process).**

| | | | |
|---|---|---|---|
| Initial denaturation | | 96°C for 5 minutes | |
| Cyclic program | | 30 cycles | |
| | Step 1 (denaturation) | | 95°C for 1 minute |
| | Step 2 (annealing) | | 54°C for 1 minute |
| | Step 3 (extension) | | 74°C for 30 seconds |
| Final extension | | 72°C for 10 minutes | |

### EXAMPLES

### Example 1:

The original blood sample was taken in the Microbiology Department of Hospital Universitario La Paz in a standard ward blood extraction format by intravenous route. The set of clinical symptoms presented by the patient required an exact identification of the pathogen because it did not allow defining the origin or progress, being subjected to prophylactic antibiotic treatment according to standard practice for diagnosed but non-characterized infections. One milliliter (1 ml) of the blood sample was inoculated into a standard hemoculture for sample enrichment, taking 7h to generate a positive result for microbial growth by incubation at 37°C. Two drops of the hemoculture were deposited on the GenoCard® system (Hain Lifescience) for the immobilization of samples from hemoculture, being adsorbed on the surface of the perforated card. Using a punch, six perforations were made to extract six pieces of adsorbed surface, which were immediately transferred to a multiwell plate at a ratio of two per well prepared as explained below.

The plate was divided into groups of three wells/containers. The reaction mixture made up of 0.4 µl of Ultratools DNA polymerase enzyme, manufactured by Biotools Biotechnological & Medical Laboratories S.A., 5 µl of the reaction buffer accompanying the aforementioned enzyme and marketed with it, between 0.1 µl and 0.3 µl of a 100 mM solution containing the four deoxyribonucleotides forming the chain of the deoxyribonucleic acid (dATP, dTTP, dGTP, dCTP), and between 0.2 µl and 0.4 µl of a 100 µM solution of the primer pair described in Table 2 amplifying the V1 region, was added in the first well of each of these groups. The stabilization mixture, made up of between 1 µl and 4 µl of a 1 M trehalose dihydrate solution, between 1 µl and 3 µl of a 0.75 M melezitose monohydrate solution, between 1 µl and 4 µl of glycogen at a concentration of 200 gr/l, and between 0.1 µl and 0.5 µl of 0.05 M DL lysine, was added to this reaction mixture. The same reaction mixture and the same stabilization mixture as those used for the first well were added in the second well, replacing the primers amplifying the V1 region with those amplifying the V2 region. The same reaction mixture and the same stabilization mixture as those used for the first well were added in the third well, replacing the primers amplifying the V1 region with those amplifying the V3 region.

The plate thus prepared was introduced in a vacuum drying oven and was subjected to a drying process by heating the plate between 30°C and 37°C and subjecting it to a vacuum of 30 millibars for a time of two to four hours, until achieving a degree of humidity between 10% and 20%, a stabilized reaction mixture containing in the same well all the elements and reagents necessary for performing the amplification reaction on the sequence of the nucleic acid to be sequenced thereby being obtained. The preceding process performed to achieve the stabilized reaction mixture can be repeated, in addition to the multiwell plate used, in any other container or reaction chamber or surface used or which may be used for performing the nucleic acid amplification reaction.

The amplification was performed under the conditions illustrated in Table 3, generating a series of amplification products that were transferred to the pyrosequencing plate according to the guideline recommended by the manufacturer of the instrument used for pyrosequencing (Sample Preparation Guidelines for PSQ™96 and PSQ 96MA Systems, prepared by Biotage AB, Sweden). Subsequent pyrosequencing was performed in the PSQ™96 apparatus, manufactured by Biotage AB, Sweden, using the enzymatic mixture for sequencing by synthesis described in the preceding sections (high-fidelity ultrapure DNA polymerase, ATP-sulfurylase, luciferase, apyrase, sequencing primer, luciferin, adenosine-5'-phosphosulfate (APS), deoxynucleotides to be incorporated in the extension reaction of the DNA chain to be sequenced (dATP, dCTP, dGTP, dTTP), and reaction buffer, generating the pyrogram shown in Figure 2 and automatically processed by IdentiFire® software (Biotage AB, Sweden) (SEQ. ID. No 7). The result of the automatic alignments according to that described in the description of the invention produced the unequivocal result with 100% identity for the pathogen ENTEROCOCCUS FAECALIS (Figure 2).

### Example 2:

The original sample was taken in the Microbiology Department of Hospital Universitario La Paz in a standard ward blood extraction format by intravenous route. The set of clinical symptoms presented by the patient required an exact identification of the pathogen because it did not allow defining the origin or progress, being subjected to prophylactic antibiotic treatment according to practice for diagnosed but non-characterized infections. A possible set of polymicrobial clinical symptoms is suspected. One milliliter (1 mi) of the blood sample was inoculated into a standard hemoculture for sample enrichment, taking 7h to generate the positive result for microbial growth by incubation at 37°C. Two drops of the hemoculture were deposited on the GenoCard® system (Hain Lifescience) for the immobilization of samples from hemoculture, being adsorbed on the surface of the perforated card. Using a punch, six perforations were made to extract six pieces of adsorbed surface, which were immediately transferred to a multiwell plate at a ratio of two per well prepared as explained below.

The plate was divided into groups of three wells/containers. The reaction mixture made up of 0.4 µl of Ultratools DNA polymerase enzyme, manufactured by Biotools Biotechnological & Medical Laboratories S.A., 5 µl of the reaction buffer accompanying the aforementioned enzyme and marketed with it, between 0.1 µl and 0.3 µl of a 100 mM solution containing the four deoxyribonucleotides forming the chain of the deoxyribonucleic acid (dATP, dTTP, dGTP, dCTP), and between 0.2 µl and 0.4 µl of a 100 µM solution of the primer pair described in Table 2 amplifying the V1 region, was added in the first well of each of these groups. The stabilization mixture, made up of between 1 µl and 4 µl of a 1 M trehalose dihydrate solution, between 1 µl and 3 µl of a 0.75 M melezitose monohydrate solution, between 1 µl and 4 µl of glycogen at a concentration of 200 gr/1, and between 0.1 µl and 0.5 µl of 0.05 M DL lysine, was added to this reaction mixture. The same reaction mixture and the same stabilization mixture as those used for the first well were added in the second well, replacing the primers amplifying the V1 region with those amplifying the V2 region. The same reaction mixture and the same stabilization mixture as those used for the first well were added in the third well, replacing the primers amplifying the V1 region with those amplifying the V3 region.

The plate thus prepared was introduced in a vacuum drying oven and was subjected to a drying process by heating the plate between 30°C and 37°C and subjecting it to a vacuum of 30 millibars for a time of two to four hours, until achieving a degree of humidity between 10% and 20%, a stabilized reaction mixture containing in the same well all the elements and reagents necessary for performing the amplification reaction on the sequence of the nucleic acid to be sequenced thereby being obtained. The preceding process performed to achieve the stabilized reaction mixture can be repeated, in addition to the multiwell plate used, in any other container or reaction chamber or surface used or which may be used for performing the nucleic acid amplification reaction.

The amplification was performed under the conditions illustrated in Table 3, generating a series of amplification products that were transferred to the pyrosequencing plate according to the guideline recommended by the manufacturer of the instrument used for pyrosequencing (Sample Preparation Guidelines for PSQ™96 and PSQ 96MA Systems, prepared by Biotage AB, Sweden). Subsequent pyrosequencing was performed in the PSQ™96 apparatus, manufactured by Biotage AB, Sweden, using the enzymatic mixture for sequencing by synthesis described in the preceding sections (high-fidelity ultrapure DNA polymerase, ATP-sulfurylase, luciferase, apyrase, sequencing primer, luciferin, adenosine-5'-phosphosulfate (APS), deoxynucleotides to be incorporated in the extension reaction of the DNA chain to be sequenced (dATP, dCTP, dGTP, dTTP), and reaction buffer, generating the pyrogram shown in Figure 3 and automatically processed by the IdentiFire® software (SEQ. ID. No. 8). The result of the automatic alignments according to that described in the detailed description of the invention produced the unequivocal result with 100% identity for the pathogen *Moraxella catarrhalis* and several potential results with varieties of a zoonotic character which reached 98% as illustrated in the final report generated by the IdentiFire^{®} system (Biotage AB, Sweden). The subsequent sub-culture and antibiogram allowed identifying at least two varieties of *Moraxella,* confirming the positive result for the *M*. *Catharrhalis* variety and the presence of the zoonotic varieties in polymicrobial infection (Figure 3).

### Example 3:

To show the enhancing effect of the pyrosequencing reaction of the mixture used for stabilization of the amplification reaction mixture (trehalose, melezitose, lysine and glycogen) by means of gelling, three blood samples were taken on the same day, and each of them was subjected to hemoculture. The three hemocultures generated a positive value in the incubator after eight hours and they were sub-cultured in non-selective agarblood plates for counting colony forming units (CFUs).

The three produced a result in the same order of dilution, so the count indicates an initial concentration in the same order of magnitude used to start and very similar after enrichment. The determination of the range of concentration of the three assayed samples was carried out by seeding dilutions up to a value of 10⁻⁹ in plates containing Mueller-Hinton agar (5% blood) and incubating at 37°C for 18h. The bacterial concentration was adjusted to the colony count in the plate corresponding to the highest dilution with the presence of bacteria. The reading was repeated at 24 h, such that the final concentration in the Mueller-Hinton medium was approximately 5 x 10⁵ CFU/ml for the three assayed samples.

Two samples randomly selected from among the three characterized by means of the process described in the preceding paragraph were processed using reaction tubes each of which containing the biotinylated primers described in Table 2 as Bio-V1 F and V1b for amplification of the V1 region of the 16S rRNA gene (SEQ. ID. No. 1 and SEQ. ID. No. 2), previously incorporated on the plate at the precise concentrations required, and stabilized as detailed in the process described in Example 1 of the present invention, together with the remaining reagents and enzymes necessary for performing the amplification reaction.

The reaction mixture made up of 0.4 µl of Ultratools DNA polymerase enzyme, manufactured by Biotools Biotechnological & Medical Laboratories S.A., 5 µl of the reaction buffer accompanying the aforementioned enzyme and marketed with it, between 0.1 µl and 0.3 µl of a 100 mM solution containing the four deoxyribonucleotides forming the chain of the deoxyribonucleic acid (dATP, dTTP, dGTP, dCTP), and between 0.2 µl and 0.4 µl of a 100 µM solution of the primer pair described in Table 2 amplifying the V1 region, was added in the wells where each of these samples was characterized. The stabilization mixture, made up of between 1 µl and 4 µl of a 1 M of trehalose dihydrate solution, between 1 µl and 3 µl of a 0.75 M melezitose monohydrate solution, between 1 µl and 4 µl of glycogen at a concentration of 200 gr/l, and between 0.1 µl and 0.5 µl of 0.05 M DL lysine, was added to this reaction mixture.

After the amplification reaction and subsequent pyrosequencing of the amplified fragment (the initial amplification product is directly transferred to the pyrosequencing plate for denaturation, equilibration and pyrosequencing, per se, without the need for intermediate quantification), a 36-base sequence (SEQ. ID. No. 9) and a 38-base sequence (SEQ. ID. No. 10) with maximum quality were obtained, using a dispensing program specific for these primers with 60 pyrosequencing cycles. The pyrograms obtained according to the IdentiFire® software of the PyroMark Q96 ID pyrosequencer manufactured by Biotage AB are shown in Figures 4A and B.

The third sample was processed in parallel by means of the same pyrosequencing process, but having performed the initial amplification without applying the gelled mixture, manually mixing, by means of a pipette, the different reagents and enzymes necessary for performing the amplification reaction in the reaction tube, including the biotinylated primers described in Table 2 as Bio-V1F and V1b for amplification of the V1 region of the 16S rRNA gene (0.4 µl of Ultratools DNA polymerase enzyme, manufactured by Biotools Biotechnological & Medical Laboratories S.A., 5 µl of the reaction buffer accompanying the aforementioned enzyme and marketed with it, between 0.1 µl and 0.3 µl of a 100 mM solution containing the four deoxyribonucleotides forming the chain of the deoxyribonucleic acid (dATP, dTTP, dGTP, dCTP), and between 0.2 µl and 0.4 µl of a 100 µM solution of the primer pair described in Table 2 and amplifying the V1 region).

After the amplification reaction and subsequent pyrosequencing applying the algorithms to calculate the quality of the sequence based on the amount of luminescence recorded by the PyroMark Q96 ID Biotage AB system, the sequence shown in Figure 4C (SEQ. ID. No 11) was obtained.

Only the first four sequenced bases were considered to have maximum quality by the IdentiFire® software of the PyroMark Q96 ID Biotage AB pyrosequencer. A quality determined as low by the software was obtained for 27 bases, and 2 bases of the total 33 bases were qualified as having intermediate quality by the IdentiFire® software of the PyroMark Q96 ID Biotage AB pyrosequencer. The pyrogram obtained is shown as an image in Figure 4C.

In the case of the non-gelled mixture, only the first three bases had optimal quality because after the third round of sequencing, the bottom starts to generate interferences with the emissions of the incorporated dNTPS.

In this Example 3 it can be observed that 100% of the sequences obtained using the gelling step are identified as optimal sequences, there being no indeterminacies, whereas only the assay that does not use stabilization by gelling only 12% of the sequence obtained is considered an unequivocal sequence, 6% of the sequence is considered as having intermediate resolution and the rest of the sequence obtained (27 out of 33 bases, i.e., 82%) is considered as possible indeterminacies.

## Claims

1. A method for taxon-specific detection, differentiation and identification of eubacteria in a biological sample by means of sequencing analysis techniques, specifically pyrosequencing of three regions of the 16S bacterial ribosomal RNA gene, **characterized in that** it comprises the following steps:
a. Stabilizing the reaction mixture by gelling by means of adding a stabilizing aqueous solution to the medium and subsequently drying to a degree of humidity between 10% and 30%.
b. PCR amplification reaction of the total DNA extracted from the sample using the primer pairs specified in Table 2 (SEQ. ID. No. 1 to 6).
c. Sequencing by synthesis, specifically pyrosequencing the PCR products obtained in step a. using the same non-biotinylated primers indicated in Table 2 and used for amplifying the total DNA extracted from the sample (SEQ. ID. No. 2, 4 and 6).

2. The method according to claim 1, **characterized in that** the amplification reaction is carried out by means of a nucleic acid (PCR) amplification reaction consisting of an initial denaturation at 95°C for 5 minutes and 30 denaturation cycles at 95°C for 1 minute, annealing at 54°C for 1 minute, extension at 74°C for 30 seconds, and a final extension at 72°C for 10 minutes.

3. The method according to claims 1-2, **characterized in that** the sequences obtained from PCR amplification are subjected to pyrosequencing and are subsequently identified by means of comparison with the sequences deposited and registered in public or private databases.

4. The method according to claims 1-3, **characterized in that** each of the sequences obtained by means of sequencing the fragments amplified by means of the amplification reaction described in claim 3 provides an additional level of information, such that if the sequence obtained by means of the reaction that was amplified using amplification primers SEQ. ID. No. 5 and 6 did not provide a sufficient level of information to assure the precise identification of the eubacteria species present in the sample, the overlap of this sequence with the sequence obtained by means of the reaction which was amplified using amplification primers SEQ. ID. No. 3 and 4 can provide precise information at the eubacterial species and genus level, and if the overlapped sequence still did not provide a sufficient level of coincidence to assure its identification, its posterior overlap with the sequence obtained by means of the reaction which was amplified using amplification primers SEQ. ID. No. 1 and 2 gives in all cases a sequence precisely informing about the family, genus and/or species of the eubacteria present in the sample.

5. The method according to claims 1-4, **characterized in that** an improvement in the pyrosequencing resolution of between 75% and 90% is obtained in the pyrosequencing reaction and as a result a sequencing of longer fragments is also obtained by using the gelling mixture formed by trehalose, melezitose, glycogen or raffinose and lysine or betaine.

6. The method according to claim 5, **characterized in that** the gelling mixture is made up of trehalose, melezitose, glycogen and lysine.

7. A kit consisting of three reaction tubes or containers, or a series of three reaction tubes, each of these tubes or containers containing all the elements necessary for performing the PCR reaction (ultrapure DNA polymerase, deoxynucleotides dATP, dCTP, dGTP, dTTP, reaction buffer and reaction primers) described in claim 1, such that the first tube contains primers SEQ. ID. No. 1 and 2, the second tube SEQ. ID. No. 3 and 4, and the third tube primers SEQ. ID. No. 5 and 6.

8. The kit according to claim 7, **characterized in that** it consists of three tubes or containers containing all the reagents necessary for carrying out the pyrosequencing reaction: high-fidelity ultrapure DNA polymerase, ATP- sulfurylase, luciferase, apyrase, sequencing primer, luciferin, adenosine-5'-phosphosulfate, deoxynucleotides dATP, dCTP, dGTP, dTTP, reaction buffer and pyrosequencing primer, such that pyrosequencing primer SEQ. ID. No. 2 is added in the first tube for the purpose of sequencing the amplicon obtained by means of the amplification performed with amplification primers SEQ. ID. No. 1 and 2, pyrosequencing primer SEQ. ID. No. 4 is added in the second tube for the purpose of sequencing the amplicon obtained by means of the amplification performed with amplification primers SEQ. ID. No. 3 and 4, and pyrosequencing primer SEQ. ID. No. 6 is added in the third tube for the purpose of sequencing the amplicon obtained by means of the amplification performed with amplification primers SEQ. ID. No. 5 and 6.

9. The kit according to claim 8, **characterized in that** each of the tubes indicated in the preceding claim are stabilized by means of adding a stabilization mixture containing trehalose, melezitose, lysine and glycogen, subsequently being dried by means of applying a vacuum at a temperature of 30°C.

10. The kit according to claims 7-9, **characterized in that** it contains all the tubes with the ingredients necessary for carrying out the method of the claims 1-6.


SEQUENCE LISTING

<110>  Biotools Biotechnological & Medical Laboratories, S.A.

<120>  COMPOSICIÓN, MÉTODO Y KIT PARA LA DETECCIÓN DE BACTERIAS MEDIANTE
        SECUENCIACIÓN

<130>  AX090153WO

<160>  11

<170>  PatentIn version 3.3

<210>  1
<211>  22
<212>  DNA
<213>  Artificial

<220>
<223>  V1F

<400>  1
gkagagtttg atcctggctc ag                                              22

<210>  2
<211>  23
<212>  DNA
<213>  Artificial

<220>
<223>  V1b

<400>  2
gyrttactca cccgtycgcc rct                                             23

<210>  3
<211>  19
<212>  DNA
<213>  Artificial

<220>
<223>  AS5

<400>  3
acacggycca gactcctac                                                  19

<210>  4
<211>  23
<212>  DNA
<213>  Artificial

<220>
<223>  AS9b

<400>  4
cggctgctgg cacgkagtta gcc                                             23

<210>  5
<211>  20
<212>  DNA
<213>  Artificial

<220>
<223>  V3F

<400>  5
gcaacgcgaa gaaccttacc                                                    20


<210>  6
<211>  21
<212>  DNA
<213>  Artificial

<220>
<223>  V3S

<400>  6
gacgacarcc atgcascacc t                                                  21


<210>  7
<211>  37
<212>  DNA
<213>  Artificial

<220>
<223>  Secuencia resultante de la reacción de pirosecuenciación llevada
       a cabo en el ejemplo 1, con un 100% de identidad para el patógeno
       Enterococcus faecalis

<400>  7
gtggctttct ggttagatac cgtcagggga cgttcag                                 37


<210>  8
<211>  42
<212>  DNA
<213>  Artificial

<220>
<223>  Secuencia resultante de la reacción de pirosecuenciación llevada
       a cabo en el ejemplo 2, con un 100% de identidad para el patógeno
       Moraxella catarrhalis

<400>  8
ggtgcttatt ctgtgggtaa cgtcagggct tatgggtatt aa                           42


<210>  9
<211>  36
<212>  DNA
<213>  Artificial

<220>
<223>  Secuencia resultante de la reacción de pirosecuenciación llevada
       a cabo en el ejemplo 3 empleando la mezcla de gelificación

<400>  9
ggtgcttatt cttcaggtac cgtcatcggc cgccga                                  36


<210>  10
<211>  38
<212>  DNA
<213>  Artificial

<220>
<223>  Secuencia resultante de la reacción de pirosecuenciación llevada
      a cabo en el ejemplo 3 empleando la mezcla de gelificación

<400>  10
gtccctttct ggtaagatac cgtcacagtg tgaacttt                              38


<210>  11
<211>  33
<212>  DNA
<213>  Artificial

<220>
<223>  Secuencia resultante de la reacción de pirosecuenciación llevada
      a cabo en el ejemplo 3 no empleando la mezcla de gelificación

<400>  11
cacagtctat ggtgtagcaa gctacggtat aaa                                   33